# DEMANDE DE BREVET EUROPEEN

(11) **EP 0 650 717 A1**
(43) Date de publication de la demande: **03.05.1995**
(21) Numéro de dépôt: 94402318.3
(22) Date de dépôt: 17.10.1994
(51) Int. Cl.: A61K 7/00, A61K 7/48

(54) **Emulsion eau dans huile contenant des polyols et son utilisation en cosmétologie**

(30) Priorité: 29.10.1993 FR 9312918
(71) Demandeur: L'OREAL, F-75008 Paris (FR)
(72) Inventeur: Griat, Jacqueline, F-94480 Ablon (FR); Lambert, Jacqueline, F-75013 (FR); Touzan, Philippe, F-31520 Ramonville Saint Agne (FR)
(74) Mandataire: Lhoste, Catherine

(57) **Abrégé**

L'invention se rapporte à une émulsion contenant une phase aqueuse dispersée dans une phase huileuse à l'aide d'un ester mixte d'acide isostéarique et d'acide succinique avec la glycérine, et au moins 10% en poids de polyol par rapport au poids total de l'émulsion.

Cette émulsion est destinée en particulier au traitement cosmétique et/ou dermatologique des mains.

## Description

La présente invention se rapporte à une émulsion eau dans huile (E/H) contenant des polyols. Cette émulsion se présente sous forme d'une crème pouvant servir de base à l'obtention d'une crème blanche ou colorée, destinée notamment au traitement cosmétique (par voie topique) du visage, du corps, des jambes et plus spécialement des mains en vue de les protéger.

Par incorporation d'actif approprié cette crème peut, en outre, servir au traitement dermatologique de mêmes zones corporelles.

Dans le domaine cosmétique, il est courant d'utiliser des crèmes constituées d'une émulsion eau dans huile renfermant en tant qu'hydratant ou humectant des polyols.

Ainsi, il est décrit dans le document JP-A-58/ 105906 une crème du type eau dans huile contenant de 1,5% à 60% en poids (par rapport au poids total de la crème) de polyol et un tensioactif hydrophobe du type ester de polyglycérine.

Par ailleurs, le document JP-A-43/60821 enseigne une crème pour le traitement topique des peaux sèches, du type eau dans huile contenant 15% à 45% en poids (par rapport au poids total de la crème) de glycérine et un émulsionnant de valeur de HLB de 8 à 11. Le HLB (hydrophilic/lipophilic balance en terminologie anglo-saxonne) représente l'équilibre entre la dimension et la force du groupe hydrophile, et la dimension et la force du groupe lipophile de l'émulsionnant.

Du fait de leur taux élevé de polyol, ces crèmes présentent l'inconvénient de conférer à la peau, après application, un caractère fortement collant comparable à celui de la glue. Ce caractère collant peut être rédhibitoire en ce qui concerne leur emploi pour les mains, par privation momentanée de l'utilisateur de ces outils précieux.

Il est par ailleurs connu du document WO-A-93/00880 l'utilisation de polyol à un taux élevé (15% à 30% en poids) dans des crèmes eau dans huile qui, bien que ne comportant pas de conservateur, présentent des propriétés anti-microbiennes. Ces crèmes présentent les mêmes inconvénients que ceux des crèmes citées précédemment.

Pour ne pas présenter ce caractère collant, les crèmes spécifiques au traitement des mains, utilisées actuellement, sont des émulsions eau dans huile contenant un faible taux de polyol (en général de l'ordre de 5%). Ces crèmes ont pour but d'adoucir, d'assouplir et de protéger la peau des mains, ainsi que de diminuer les rougeurs et/ou les gerçures. Des exemples de crèmes pour les mains sont notamment décrits dans le document JP-A-43/70152.

Malheureusement, ces crèmes présentent une efficacité très relative, ne permettant pas le traitement des crevasses et laissant les mains sèches.

En terme d'efficacité, la crème de référence pour le traitement des mains, actuellement sur le marché, est la crème NEUTROGENA (R) "Formule Norvégienne", "Concentré" contenant de la glycérine. Cette crème filmogène, particulièrement efficace contre les rougeurs et gerçures, présente malgré tout les inconvénients d'une texture et d'un aspect peu attrayants, d'une pénétration et d'un assouplissement de la peau insuffisants. De plus les mains, même après traitement, sont encore trop sèches.

Il subsiste donc le besoin d'une crème de soin des mains ne présentant pas de caractère collant, ayant une bonne pénétration et un effet anti-desséchant remarquable, et permettant de diminuer les rougeurs, les gerçures voire même les crevasses.

L'invention a justement pour objet une émulsion eau dans huile approprié au traitement cosmétique et/ou dermatologique des mains. Cette émulsion peut aussi servir au traitement du visage, du corps et des jambes.

De façon plus précise, l'invention se rapporte à une émulsion contenant une phase aqueuse dispersée dans une phase huileuse à l'aide d'un émulsionnant, caractérisée en ce que cet émulsionnant est formé essentiellement d'un ester mixte d'acide isostéarique et d'acide succinique avec la glycérine et en que l'émulsion contient, en outre, au moins 10% en poids de polyol par rapport au poids total de l'émulsion.

Cette émulsion à haut taux de polyol peut être utilisée avantageusement pour le traitement des mains. Elle confère de façon surprenante un effet collant quasi inexistant sur les mains. En outre, du fait de son taux élevé de polyol, elle pénètre efficacement dans la peau et assure notamment une bonne hydratation et une grande souplesse de celle-ci, ainsi qu'une pénétration élevée dans la peau conduisant à une réduction des rougeurs et des gerçures.

Il est certes connu par le document FR-A-2686510 d'utiliser un ester mixte d'acide isostéarique et d'acide succinique avec la glycérine comme émulsionnant dans une émulsion eau-dans-huile, mais cet émulsionnant est nécessairement associé à une émulsionnant siliconé. En effet, le problème que cherchait à résoudre l'objet de ce document était l'incorporation de quantités élevées de silicones.

Selon l'invention le taux de polyol peut représenter jusqu'à 40% en poids par rapport au poids total de l'émulsion et de préférence être choisi de 12% à 30%, et par exemple de 12% à 20%.

Comme polyol utilisable dans l'invention, on peut citer la glycérine, le mannitol, le sorbitol, le glucose et dérivé de sucre ainsi que la diglycérine. Ces polyols peuvent être utilisés seuls ou en mélange.

Il est connu du document US-A-4089879 des émulsions eau dans huile utilisables en cosmétologie, contenant un ester mixte d'acide isostéarique et d'acide succinique avec de la glycérine comme émulsionnant et un polyol (glycérine) à un taux faible de 3% en poids par rapport au poids total de l'émulsion.

Ce faible taux de polyol ne procurerait pas, en cas d'utilisation de ces émulsions sur les mains, la souplesse, l'hydratation, l'effet anti-desséchant recherchés, ni une pénétration suffisante de cette émulsion dans la peau.

De plus, ce document ne suggère pas d'augmenter la quantité de glycérine pour obtenir ces propriétés.

Enfin, une émulsion eau dans huile stable est très difficile à réaliser et est un choix judicieux des différents constituants dans des proportions bien définies. Aussi, un simple changement de produit ou de concentration peut casser l'émulsion.
L'estérification de la glycérine dans l'émulsionnant selon l'invention peut être partielle ou totale. Elle peut être réalisée comme décrit dans le document US-A-4089879. En particulier, l'ester mixte utilisé est celui vendu par la Société HULS, sous le nom de Imwitor 780 K et correspondant à l'isostéaryl diglycéryl succinate (selon la nomenclature CTFA).

Cet émulsionnant présente l'avantage d'être stable à l'oxydation, de ne pas rancir, d'être non toxique et parfaitement compatible avec la peau.

L'association de cet émulsionnant avec un taux élevé de polyol confère à l'émulsion la plupart de ses propriétés.

L'émulsionnant est utilisé de préférence à un taux de 0,5% à 8% en poids par rapport au poids total de l'émulsion et mieux à un taux de 2% à 5% en poids.

En vue d'améliorer les propriétés cosmétiques de l'émulsion, il est avantageux d'utiliser dans la phase huileuse des silicones volatiles, seules ou en mélange, comme par exemple les silicones linéaires comportant de 1 à 4 groupements 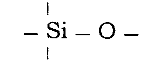
comme l'hexaméthyldisiloxane et l'octaméthyltrisiloxane ; les silicones cycliques comme l'octaméthylcyclotetra siloxane et la decaméthylcyclopenta siloxane (appelées cyclodiméthicones selon la nomenclature CTFA).

Il est toutefois possible d'utiliser d'autres huiles comme les huiles végétales (jojaba, abricot), minérales (huile de vaseline), ou de synthèse (huile de purcelles), ou encore les huiles fluorées (perfluoropolyéther).

La phase huileuse représente de 10% à 45% en poids par rapport au poids total de l'émulsion et de préférence de 20% à 30%.
En outre, les silicones peuvent représenter tout ou partie de la phase huileuse. Les autres huiles ne représentent, en général, que de 1% à 10% en poids par rapport au poids total de la phase huileuse.

Afin d'assurer une bonne conservation dans le temps de l'émulsion, il est possible d'y incorporer un système conservateur contenant un ou plusieurs composés, par exemple choisis parmi les parahydroxybenzoates d'alkyle (nom CTFA), les libérateurs de formol (imidazolidinyluree), la chlorphenesine, ...

Le système conservateur peut représenter de 0,1% à 1% en poids par rapport au poids total de l'émulsion.

Il est en outre possible d'ajouter à l'émulsion d'autres adjuvants comme les parfums, les gélifiants lipophiles (Bentone, cire, gomme) ou hydrophiles (chitosane), les matières colorantes, des actifs, et tous les adjuvants classiquement utilisés dans les domaines cosmétique et dermatologique.

Comme actifs, on peut utiliser des actifs hydrophiles comme les protéines ou hydrolysat de protéine (restructurant), l'allantoïne (anti-inflammatoire), les eaux florales (adoucissant), chitosane (humectant), les acides aminés et panthénol (hydratant, apaisant), les α-hydroxyacides, l'urée ainsi que des actifs lipophiles comme les vitamines A et E et leurs esters (régénérant, protecteur), l'α-bisabolol (anti-inflammatoire), les céramides (pour cohésion cellulaire), les acides gras essentiels (nourrissant).

On peut aussi utiliser des filtres UV à propriété lipophile ou hydrophile. Les matières colorantes peuvent être soit des pigments (oxyde de fer, de titane), soit des colorants organiques (sel di-sodique de Ponceau).

Comme indiqué précédemment, l'émulsion de l'invention peut avantageusement être utilisée pour le traitement dermatologique et cosmétique du visage et du corps humain et plus spécialement des mains.

Pour le visage, l'émulsion de l'invention peut servir de base pour former par exemple une crème de soin, une crème solaire ou encore un masque hydratant.

Aussi, l'invention a encore pour objet une composition cosmétique formée d'une émulsion telle que définie précédemment ainsi que l'utilisation de cette émulsion pour le traitement cosmétique des mains.

L'invention a encore pour objet une utilisation de cette émulsion pour la préparation d'une crème destinée au traitement dermatologique des mains.

D'autres caractéristiques et avantages de l'invention ressortiront mieux de la description qui va suivre, donnée à titre illustratif et non limitatif.

L'émulsion de l'invention est obtenue, tout d'abord, en introduisant dans la phase huileuse l'ester mixte d'acide gras, contenant éventuellement les composés lipophyles (actifs, parfums, colorants gélifiants), puis en homogénéisant l'ensemble à 40°C ; parallèlement, on dissout à température ambiante les polyols dans de l'eau éventuellement déminéralisée, puis les conservateurs et les actifs hydrophiles. Ensuite, on introduit la phase aqueuse dans la phase huileuse, toujours à 40°C, sous une forte agitation qui est maintenue pendant 10 minutes environ. Enfin, on refroidit l'ensemble à 20°C.

On obtient ainsi une crème stable, lisse et brillante, d'un bel aspect translucide.

Dans les exemples ci-après, toutes les crèmes obtenues sont légères, translucides, d'odeur agréable et non collante après application. En outre, les quantités sont données en % en poids.

### . Exemple 1 : Crème de soin des mains réparatrice et protectrice

### Phase lipophile

- Imwitor 780 K (Isostéaryl diglycéryl succinate) 3
- Octyldodécanol 4
- Octylméthoxycinnamate (Parsol McX vendu par Givaudan) (filtre) 2
- Quaternium-18 Hectorite (Bentone) (gélifiant) 2
- Cyclométhicone (silicone volatile) 20
- Parfum 0,2

### Phase hydrophile

- Glycérine 10
- Sorbitol 5
- Conservateurs 0,5
- Protéine végétale (restructurant) 0,2
- Eau déminéralisée qsp 100

### . Exemple 2 :

L'émulsion de cet exemple se différencie de celle de l'exemple 1 par l'introduction d'oxyde de fer à une concentration de 0,0024% en poids afin de colorer légèrement en rose la crème.

### . Exemple 3 : Crème de soin du visage nutritive et protectrice

### Phase lipophile

- Huile d'avocat 7
- Imwitor 780 K 5
- Cyclométhicone 13
- Octyldodécanol 3
- céramide de synthèse 0,05
- Mélange de Cyclométhicone et diméthicone 6 (gomme de silicone)
- Parsol MCX 2
- Parfum 0,3

### Phase hydrophile

- Collagène marin (adoucissant) 0,5
- Proline (régénérant) 1
- Mannitol 5
- Glycérine 12
- Chitosane PCA (Kytamer PC, de chez Armechol) (gélifiant) 0,2
- Conservateurs 0,5
- Eau qsp 100

### . Exemple 4 : Crème solaire visage et corps protectrice et anti-desséchante

### Phase lipophile

- Cyclométhicone 20
- Parsol MCX 5
- Dioxyde de titane (charge filtrante) 3
- Beurre de karité (huile) 5
- Imwitor 780 K 4
- Bentone (gélifiant) 3
- Parfum 0,3
- Conservateur 0,2

### Phase hydrophile

- Colorants 0,1
- Glycérine 15
- Conservateurs 0,4
- Eau qsp 100

### . Exemple 5 : Créme corporelle hydratante et nutritive

### Phase lipophile

- Imwitor 780 K 4
- Octyl dodecanol 4
- Huile d'abricot 8
- Cyclométhicone 12
- Céramide de synthèse 0,1
- Gomme de silicone 6

### Phase hydrophile

- Conservateurs 1
- Glycérine 12
- Mannitol 8
- Amidon réticulé (Dry-Flow + de National Starch) 0,3 (matifiant- adoucissant)
- Chitosan PCA (gélifiant) 0,2
- Urée (hydratant) 1
- Eau déminéralisée qsp 100

### . Exemple 6 : Crème de soin des mains réparatrice et protectrice

### Phase lipophile

- Imwitor 780 K (Isostéaryl diglycéryl succinate) 3
- Octyldodécanol 4
- Octylméthoxycinnamate (Parsol McX vendu par Givaudan) (filtre) 2
- Quaternium-18 Hectorite (Bentone) (gélifiant) 2
- Cyclométhicone (silicone volatile) 20
- Parfum 0,2

### Phase hydrophile

- Glycérine 35
- Mannitol 5
- Conservateurs 0,5
- Eau déminéralisée qsp 100

La crème obtenue présente un effet collant quasi-inexistant malgré les 40% de polyols.

La crème pour les mains de l'exemple 2 ainsi que la crème NEUTROGENA pour les mains ont été testées en parallèle pendant deux semaines sur deux panels similaires respectivement de 18 personnes et 11 personnes.
Il s'agissait de femmes aux mains très abîmées en raison de leurs conditions de travail d'une part et du climat rigoureux d'autre part.
Elles présentaient pour environ 1/3 d'entre elles, soit des rougeurs, soit des gerçures et parfois les deux.
La face interne des mains était souvent très altérée.

La crème de l'invention a obtenu de meilleurs scores cosmétiques que la crème NEUTROGENA en ce qui concerne :
- La pénétration : 94% d'avis favorables au lieu de 59%
- La texture : 89% d'avis favorables au lieu de 65%

De plus, la crème de l'invention a obtenu 100% d'avis favorables en ce qui concerne son étalement et 2 personnes seulement ont mentionné la présence d'un film gras sur les mains. La présence d'un film cireux est assimilé à un effet protecteur et n'a donc pas une connotation négative.

En ce qui concerne l'efficacité, ces deux crèmes ont été jugées équivalentes pour la protection contre le froid et l'eau, et pour la douceur des mains.

En revanche, la crème de l'invention a été jugée supérieure en ce qui concerne la souplesse de la peau (72% de "Satisfaites" contre 41%) et l'effet anti-desséchant (83 % de "Satisfaites" contre 47%).

Pour les personnes ayant des gerçures et des crevasses, la crème de l'invention a été reconnue comme plus efficace que la crème NEUTROGENA.

En conclusion, la crème de l'invention a été jugée comme alliant un agrément cosmétique supérieur à celui de la crème NEUTROGENA avec une action traitante et une efficacité plus performantes. Or jusqu'à ce jour, la crème NEUTROGENA pour les mains était considérée comme la plus efficace pour le soin des mains.

Par ailleurs, la crème de l'exemple 2 a été soumise à un test auprès du Public sur une trentaine de femmes dont les mains étaient sèches ou très sèches et qui présentaient, pour la plupart d'entre elles, des problèmes du type gerçures, rougeurs, desquamations ou simplement des stries de déshydratation.

Après 3 semaines d'utilisation, la majeure partie de ces personnes a pu observer une nette amélioration de l'état de leurs mains. La peau est moins sèche, plus douce, plus souple de façon importante pour 65% de l'échantillon.

Les effets protecteur et hydratant sont ressentis de manière moins importante mais se situent tout de même au-dessus de la moyenne et arrivent à un total respectif de 51% à 56% ("Très important" ou "Important").

En outre, les 3/4 des personnes ayant des gerçures ou crevasses ont perçu une atténuation de leur problème, dont la majeure partie l'évalue comme très importante ou importante.

Les propriétés cosmétiques de cette crème ont été très appréciées : facile à appliquer, légère, teneur en gras bien équilibrée.

Le confort est bon et persistant, et l'odeur agréable.

Les 3/4 environ des utilisatrices sont donc satisfaites de cette crème et l'achèteraient pour remplacer leur produit habituel.

Le motif presque exclusif de satisfaction est l'efficacité de cette crème pour les mains.

Son agrément cosmétique ne fait qu'ajouter un élément supplémentaire au plaisir de l'utiliser.

## Revendications

1. Emulsion eau dans huile, contenant une phase aqueuse dispersée dans une phase huileuse à l'aide d'un émulsionnant, caractérisé en ce que cet émulsionnant est formé essentiellement d'un ester mixte d'acide isostéarique et d'acide succinique avec la glycérine et en ce que l'émulsion contient, en outre, au moins 10 % en poids de polyol par rapport au poids total de l'émulsion.

2. Emulsion selon la revendication 1, caractérisée en ce que la phase huileuse contient des silicones volatiles.

3. Emulsion selon la revendication 1 ou 2, caractérisée en ce que la phase huileuse représente de 10% à 45% en poids par rapport au poids total de l'émulsion.

4. Emulsion selon la revendication 2 ou 3, caractérisée en ce que la phase huileuse contient au moins une huile non siliconée représentant de 1 % à 10 % en poids par rapport au poids total de la phase huileuse.

5. Emulsion selon l'une quelconque des revendications précédentes, caractérisée en ce que le polyol représente jusqu'à 40 % en poids par rapport au poids total de l'émulsion.

6. Emulsion selon l'une quelconque des revendications 1 à 5, caractérisée en ce que le polyol représente de 10 % à 20 % en poids par rapport au poids total de l'émulsion.

7. Emulsion selon l'une quelconque des revendications précédentes, caractérisée en ce que le polyol est au moins un composé choisi parmi la glycérine, la mannitol et le sorbitol.

8. Emulsion selon l'une quelconque des revendications précédentes, caractérisée en ce que le polyol est la glycérine.

9. Emulsion selon l'une quelconque des revendications précédentes, caractérisée en ce que l'ester mixte représente de 0,5 % à 8 % en poids par rapport au poids total de l'émulsion.

10. Emulsion selon l'une quelconque des revendications précédentes, caractérisée en ce que l'ester mixte représente de 2 % à 5 % en poids par rapport au poids total de l'émulsion.

11. Emulsion selon l'une quelconque des revendications précédentes, caractérisée en ce qu'elle contient, en outre, au moins un adjuvant choisi parmi les parfums, les actifs hydrophiles et lipophiles, les gélifiants des phases aqueuse et huileuse, les matières colorantes, les conservateurs.

12. Emulsion selon la revendication 10, caractérisée en ce que l'adjuvant représente de 0% à 5% en poids par rapport au poids total de l'émulsion.

13. Composition cosmétique, caractérisée en ce qu'elle consiste en une émulsion selon l'une quelconque des revendications précédentes.

14. Utilisation d'une émulsion selon l'une quelconque des revendications 1 à 12 pour le traitement cosmétique des mains.

15. Utilisation d'une émulsion selon l'une quelconque des revendications 1 à 11 pour la préparation d'une crème destinée au traitement dermatologique des mains.
